# EUROPEAN PATENT APPLICATION

(11) **EP 3 789 092 A1**
(43) Date of publication of application: **10.03.2021**
(21) Application number: 19196077.2
(22) Date of filing: 08.09.2019
(51) Int. Cl.: A63F 13/655, A61F 5/58, A63F 13/80, A63F 13/92, G09B 19/04, G16H 20/30

(54) **METHOD OF TRAINING ORAL MOTOR FUNCTION IN A HANDHELD DEVICE**

(71) Applicant: Eikelberg, Catja, 79100 Freiburg (DE)
(72) Inventor: Eikelberg, Catja, 79100 Freiburg (DE)
(74) Representative: Meyer zu Bexten, Elmar

(57) **Abstract**

Problem

The invention seeks an alternative to known methods of training oral motor function which provides the same or similar effects or is more cost-effective.

Solution

Method of training, such as for preventing or treating speech impediment, oral motor function of a user in a handheld device (10),
characterized in
capturing a live view of a camera facing the user;
augmenting the view with game elements such as virtual objects;
based on the view, tracking a mouth expression of the user while mirroring the augmented view on a display;
by means of the game elements, playfully inciting the user to vary the mouth expression.

## Description

### Technical Field

The invention relates to the products and process as per the first portion of the independent claims.

### Background Art

Speech-language pathology, sometimes referred to as logopedics, is a field of expertise practiced by a clinician known as a speech-language pathologist or therapist. In recent years, mobile applications have emerged claiming to provide support for children with speech and language impairments, such as through autonomous training at home (cf. PTL1 and NPL1).

### Summary of invention

The invention as claimed is hereinafter disclosed in such a way that the technical problem with which it deals can be appreciated and the solution can be understood.

### Technical Problem

The invention seeks an alternative to known methods of training oral motor function which provides the same or similar effects or is more cost-effective.

### Solution to Problem

The problem is solved by a system as per the second portion of Claim 1.

### Advantageous effect of invention

A method as per the invention encourages children to have fun while exploring their oral motor skills in an augmented reality (AR). To prevent speech impediments or misaligned teeth from developing, children as young as four years of age are enabled to strengthen their oral musculature and improve their awareness preventively or accompanied by speech therapy.

Additional applications include adult treatment, speech training such as for actors or speakers, or instructing the deaf in the use of lip movement.

### Brief description of drawings

Figure 1 is a schematic view of a smartphone running an app.
Figure 2 is an in-game graphic of a minigame played by a child.
Figure 3 is a flowchart of the gameplay.

### Description of embodiments

A preferred embodiment takes the form of an application (app) for a smartphone having a user-facing ("selfie") camera, display, and means which, in the context of the invention, provide generic data processing functions.

When executed, the app captures a live view of the user-facing camera. If the smartphone is being operated in the conventional posture, this view will include the face of the user looking at the display screen which is usually right below the camera. Hereinafter the user is assumed to be a child.

The app augments the view of the child's face with game elements such as virtual objects associated with one among various minigames. (Such minigame may be selected, for instance, by scanning a QR or other barcode printed in a children's book, or simply picked from a list of options on-screen.)

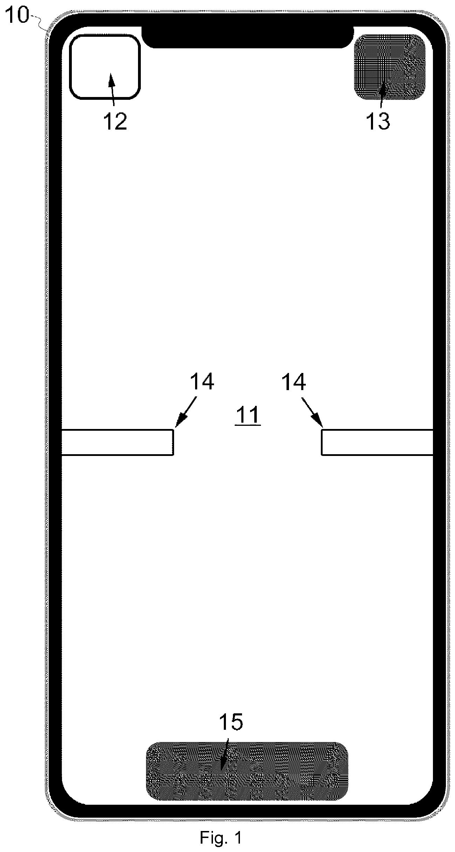

Additionally, the augmented view is overlaid with user interface elements, for example, a pause button (12), time indication (13), alignment aids (14) on mouth level, or a score (15). Rather than numerically, the latter is ideally represented through stars or other symbols to accommodate users at preschool age. Figure 1 shows an example of such screen layout.

While mirroring the augmented view on the display, the app (11) tracks the child's mouth expression, differentiating mouth poses such as "open jaw", "closed jaw", "puckered lips", or "fish gape". Depending on the minigame being played, the child is incited by means of the game elements to purposefully use one or more of these poses.

### Example

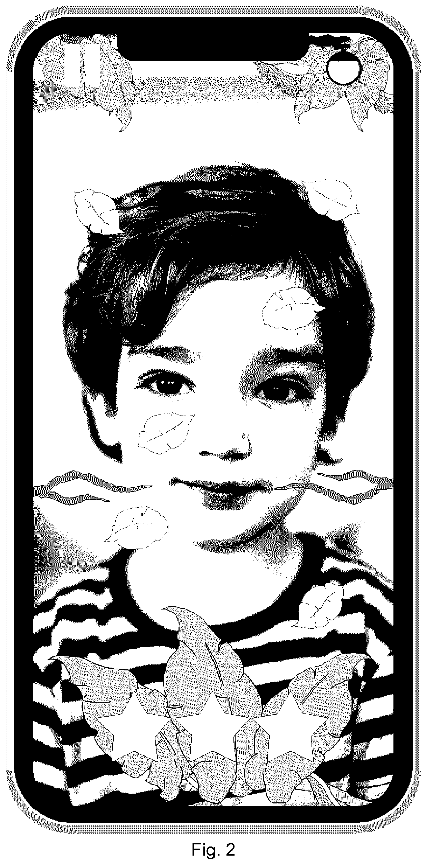

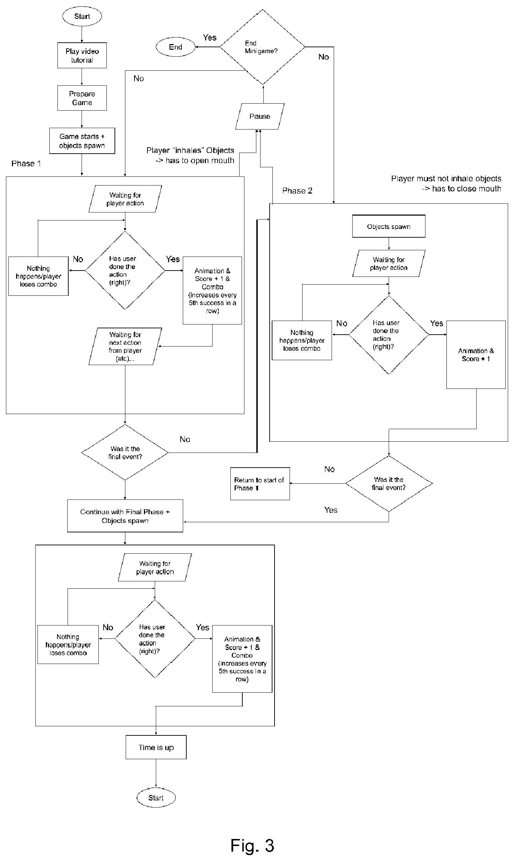

In a minigame entitled "spring-cleaning" and depicted in Figure 2, the aim is to selectively inhale virtual leaves which appear to be falling in front of the child's face. The gameplay may be gathered from the diagrammatic representation of Figure 3.

### Industrial applicability

The invention is applicable, among others, throughout the medical and therapeutic industries.

### Reference signs list

- 10: Smartphone, device
- 11: Application, computer program
- 12: Pause button
- 13: Time indication
- 14: Alignment aids
- 15: Score

### Citation list

The following documents are cited hereinbefore.

### Patent literature

PTL1: US 2012116772 A (JONES KEVIN [US]; SRIVASTAVA GARIMA [US]; AVENTUSOFT LLC [US]) 05.10.2012

### Non-patent literature

NPL1: ROCHA, Tânia, et al. The AppVox mobile application, a tool for speech and language training sessions. Expert Systems. 2019, vol.36, no.3, p.e12373.

## Claims

1. Method of training, such as for preventing or treating speech impediment, oral motor function of a user in a handheld device (10),
**characterized in**
capturing a live view of a camera facing the user;
augmenting the view with game elements such as virtual objects;
based on the view, tracking a mouth expression of the user while mirroring the augmented view on a display;
by means of the game elements, playfully inciting the user to vary the mouth expression.

2. Method as per Claim 1
wherein
the capturing, augmenting, and tracking is effected by means of a software framework, such as ARKit or ARCore, of the device (10), the framework preferably being accessed through a cross-platform game engine such as Unity.

3. Method as per any of the preceding claims
wherein
the tracking differentiates specified poses such as open, close, pucker, or gape.

4. Method as per any of the preceding claims
comprising
demonstrating the oral motor function by means of an animation, such as of a cartoon character or avatar, shown on the display.

5. Method as per any of the preceding claims
wherein
the user is incited to vary the mouth expression rhythmically, such as in sync with music being played back.

6. Method as per any of the preceding claims
comprising
playback of video footage such as for parental education.

7. Method as per any of the preceding claims
wherein
the game elements are associated with various minigames, for example, inhaling leaves or shooing butterflies.

8. Method as per Claim 7
wherein
the minigames are selected by means of a barcode such as a QR code printed in a children's book.

9. Method as per Claim 7 or Claim 8
wherein,
during the minigames, the augmented view is overlaid with user interface elements, for example, a pause button (12), time indication (13), alignment aids (14) such as on mouth level, or score (15), preferably displayed symbolically.

10. Method as per any of the preceding claims
comprising
notifying push messages, such as linking to video clips.

11. Handheld device (10), for example, smartphone (10),
**characterized in**
a front camera, display, and means adapted to execute the steps of the method as per any of the preceding claims.

12. Device (10) as per Claim 11
wherein
the camera is adapted for depth perception, such as by means of stereo vision or an infrared emitter and sensor.

13. Device (10) as per Claim 11 or Claim 12

14. Computer program (11) comprising instructions to cause the device (10) of Claim 11 to execute the steps of the method of Claim 1.

15. Computer-readable medium having stored thereon the computer program (11) of Claim 14.

## Amended claims

### Amended claims in accordance with Rule 137(2) EPC.

1. Method of training, such as for preventing or treating speech
impediment, oral motor function of a user in a handheld device (10), comprising
capturing a live view of a camera facing the user and
playfully inciting the user to vary the mouth expression,
**characterized in**
augmenting the view with game elements associated with various minigames selected by means of a barcode such as a QR code and,
based on the view, tracking a mouth expression of the user while mirroring the augmented view on a display, the user being incited by means of the game elements.

2. Method as per Claim 1
wherein
the capturing, augmenting, and tracking is effected by means of a software framework, such as ARKit or ARCore, of the device (10), the framework preferably being accessed through a cross-platform game engine such as Unity.

3. Method as per any of the preceding claims
wherein
the tracking differentiates specified poses such as open, close, pucker, or gape.

4. Method as per any of the preceding claims
comprising
demonstrating the oral motor function by means of an animation, such as of a cartoon character or avatar, shown on the display.

5. Method as per any of the preceding claims
wherein
the user is incited to vary the mouth expression rhythmically, such as in sync with music being played back.

6. Method as per any of the preceding claims
comprising
playback of video footage such as for parental education.

7. Method as per any of the preceding claims
wherein,
during the minigames, the augmented view is overlaid with user interface elements, for example, a pause button (12), time indication (13), alignment aids (14) such as on mouth level, or score (15), preferably displayed
symbolically.

8. Method as per any of the preceding claims
comprising
notifying push messages.

9. Handheld device (10), for example, smartphone (10),
**characterized in**
a front camera, display, and means adapted to execute the steps of the method as per any of the preceding claims.

10. Device (10) as per Claim 9
wherein
the camera is adapted for depth perception, such as by means of stereo vision or an infrared emitter and sensor.

11. Computer program (11) comprising instructions to cause the
device (10) of Claim 10 to execute the steps of the method of Claim 1.

12. Computer-readable medium having stored thereon the computer program (11) of Claim 11.
